# EUROPÄISCHE PATENTSCHRIFT

(11) **EP 2 164 460 B1**
(45) Veröffentlichungstag und Bekanntmachung des Hinweises auf die Patenterteilung: **09.08.2017**
(21) Anmeldenummer: 08760350.2
(22) Anmeldetag: 02.06.2008
(51) Int. Cl.: A61K 9/00, A61K 9/20, A61K 47/26

(54) **PHARMAZEUTISCHE FORMULIERUNG FÜR DIE HERSTELLUNG VON SCHNELL ZERFALLENDEN TABLETTEN**
PHARMACEUTICAL FORMULATION FOR THE PRODUCTION OF RAPIDLY DISINTEGRATING TABLETS
PRÉPARATION PHARMACEUTIQUE POUR PRODUIRE DES COMPRIMÉS À DÉLITEMENT RAPIDE

(30) Priorität: 06.06.2007 EP 07109717
(43) Veröffentlichungstag der Anmeldung: 24.03.2010
(73) Patentinhaber: BASF SE, 67056 Ludwigshafen am Rhein (DE)
(72) Erfinder: KOLTER, Karl, 67117 Limburgerhof (DE); SCHÖNHERR, Michael, 67227 Frankenthal (DE); GEBERT, Silke, 67269 Grünstadt (DE); MEYER-BÖHM, Kathrin, 90537 Feucht (DE); MASCHKE, Angelika, 68159 Mannheim (DE)
(74) Vertreter: BASF IP Association
(86) Internationale Anmeldenummer: PCT/EP2008/056763
(87) Internationale Veröffentlichungsnummer: WO 2008/148731

(56) Entgegenhaltungen:
- WO-A-98/22094
- WO-A-03/041683
- WO-A-2007/071581
- US-A1- 2002 071 864
- US-A1- 2005 244 343
- US-A1- 2005 244 492

## Beschreibung

Die vorliegende Erfindung betrifft pharmazeutische Formulierungen in Form von Kompaktaten für die Herstellung von schnell zerfallenden Tabletten, enthaltend Zucker oder Zuckeralkohole, Sprengmittel und wasserunlösliche Polymere.

Im Mund schnell zerfallende und/oder sich schnell auflösende Tabletten gewinnen für die orale Applikation von Arzneistoffen immer größere Bedeutung. Solche Tabletten müssen innerhalb kurzer Zeit, am besten innerhalb von 30 Sekunden in der Mundhöhle zerfallen, angenehm schmecken und dürfen kein sandiges Gefühl hinterlassen. Ferner sollen sie einfach herstellbar sein, wobei die Direkttablettierung erhebliche Vorteile gegenüber der Feuchtgranulation bietet, und eine hohe mechanische Festigkeit besitzen, damit sie Verpackungsprozeduren, Transporte und auch das Herausdrücken aus Verpackungen unbeschadet überstehen.

Die bisher beschriebenen Produkte und Verfahren erfüllen diese Anforderungen nicht oder nur sehr unzureichend.

Schnell zerfallende Tabletten bestehen häufig aus Zucker und Zuckeralkoholen, Brausesystemen, mikrokristalliner Cellulose und anderen nicht wasserlöslichen Füllstoffen Calciumhydrogenphosphat, Cellulose-Derivaten, Maisstärke, oder Polypeptiden. Weiterhin kommen wasserlösliche Polymere, übliche Sprengmittel (quervernetztes PVP, Na- und Ca-Salz der quervernetzten Carboxymethylcellulose, Na-Salz der Carboxymethylstärke, niedrigsubstituierte Hydroxypropylcellulose L-HPC) und im wesentlichen anorganische wasserunlösliche Bestandteile (Kieselsäuren, Silikate, anorganische Pigmente) zum Einsatz. Weiterhin können die Tabletten auch Tenside enthalten.

In der WO 2003/051338 ist eine direkttablettierbare und gut verpressbare Hilfsstoffformulierung, welche Mannit und Sorbit enthält, beschrieben. Zunächst wird durch Lösen von Mannit und Sorbit in Wasser und anschließender Sprühtrocknung (gewöhnliche Sprühtrocknung und SBD-Verfahren) eine Hilfsstoffvormischung hergestellt. Dieser coprozessierten Mischung kann zusätzlich Mannit zugesetzt werden. Tabletten, welche zusätzlich Sprengmittel, Trennmittel, Pigment und einen Wirkstoff enthalten, sollen innerhalb von 60 Sekunden in der Mundhöhle zerfallen.

In der US 2002/0071864 A1 wird eine Tablette beschrieben, welche innerhalb von 60 Sekunden in der Mundhöhle zerfällt, und hauptsächlich aus einer physikalischen Mischung von sprühgetrocknetem Mannit und einem grobkörnigen quervernetztem Polyvinylpyrrolidon sowie einer begrenzten Auswahl an Wirkstoffen formuliert ist. Diese Tabletten besitzen eine Bruchfestigkeit von ca. 40N und erzeugen ein unangenehmes, sandiges Mundgefühl.

Gemäß der US 6,696,085 B2 soll ein Methacrylsäure-Copolymer Typ C als Zerfallsmittel eingesetzt werden. Das Methacrylsäure-Copolymer Typ C ist ein magensaftresistentes Polymer, welches im sauren pH-Bereich nicht löslich ist, im pH-Bereich von 7, wie er in der Mundhöhle vorliegt, aber wasserlöslich ist. Die Tabletten weisen neben einer niedrigen Bruchfestigkeit (<20N) eine hohe Friabilität (>7%) auf und beinhalten einen hohen Anteil im Bereich von 15Gew.-% eines grobkörnigen Sprengmittels. Sie besitzen folglich eine niedrige mechanische Festigkeit und erzeugen aufgrund des hohen Anteils an grobkörnigem Sprengmittel ein unangenehmes, sandiges Mundgefühl.

EP 0839526 A2 beschreibt eine pharmazeutische Darreichungsform bestehend aus einem Wirkstoff, Erythrit, kristalliner Cellulose und einem Sprengmittel. Weiterhin wird Mannit eingearbeitet und als Sprengmittel quervernetztes Polyvinylpyrrolidon verwendet, sodass eine physikalische Mischung entsteht. Die Tabletten sollen innerhalb von 60 Sekunden in der Mundhöhle zerfallen.

In der Anmeldung JP 2004-265216 wird eine im Mund innerhalb von 60 Sekunden zerfallende Tablette, bestehend aus einem Wirkstoff, einem wasserlöslichen Polyvinylalkohol-Polyethylenglykol-Copolymer, Zucker/Zuckeralkohol (Mannit) und Sprengmittel, beschrieben.

Aufgabe der vorliegenden Erfindung war es, im Mund schnell zerfallende Tabletten und Verfahren zu deren Herstellung zu finden, die ein angenehmes Mundgefühl hinterlassen, mechanisch sehr stabil sind und eine gute Gehaltseinheitlichkeit aufweisen.

Demgemäß wurde eine pharmazeutische Zubereitung für die Herstellung von im Mund schnell zerfallenden Tabletten gefunden, welche aus Kompaktaten bestehen, erhältlich aus
A) einem agglomerierten Hilfsstoffanteil aus
   a1)60 - 97 Gew.-% mindestens eines Zuckers oder Zuckeralkohols oder Mischungen davon,
   a2)1 - 25 Gew.-% eines Sprengmittels ausgewählt aus der Gruppe bestehend aus quervernetztem Polyvinylypyrrolidon, Croscarmellose, quervernetzter Natriumcarboxymethylstärke und L-Hydroxypropylcellulose,
   a3)1 - 15 Gew.-% von wasserunlöslichen Polymeren,
   a4)0 - 15 Gew.-% wasserlöslichen Polymeren, und
   a5)0 - 15 Gew.-% weiterer pharmazeutisch üblichen Hilfsstoffen,
   , wobei die Summe der Komponenten a1) bis a5) 100 Gew.-% beträgt,
B) mindestens einem pharmazeutischen Wirkstoff,
C) 0 bis 10% Gew.-%, bezogen auf die Gesamtmenge an A) bis D), eines Schmiermittels oder Formtrennmittels,
D) gegebenenfalls weiteren pharmazeutischen Hilfssstoffe.

Weiterhin wurde ein Verfahren zur Herstellung von solchen Kompaktaten gefunden, welches dadurch gekennzeichnet ist, dass man, den agglomerierten Hilfsstoffanteil A) mit B) mindestens einem Wirkstoff, C) Schmiermittel und D) gegebenenfalls weiteren Hilfsstoffen in einem Kompaktor verarbeitet.

Weiterhin wurden im Mund schnell zerfallende Tabletten, erhältlich aus solchen Kompaktaten , gefunden. Die Tabletten zerfallen im Mund oder in wässrigem Milieu innerhalb von 60 Sekunden, bevorzugt innerhalb von 30 Sekunden, besonders bevorzugt innerhalb von 20 Sekunden. "Die Tabletten weisen bei 37 °C in Phospatpuffer, pH 7,2, eine Zerfallszeit von < 60 Sekunden auf.Die Zerfallszeit wird im Zerfallstester nach USP oder Pharm. Eur. bestimmt."

Die pharmazeutischen Zubereitungen enthalten als Komponente a1) 60 bis 97 Gew.-%, bevorzugt 70 bis 95 Gew.-%, besonders bevorzugt 75 bis 93 Gew.-% eines Zuckers, Zuckeralkohols oder Mischungen davon. Als Zucker oder Zuckeralkohole eignen sich Trehalose, Mannit, Erythrit, Isomalt, Maltit, Lactit, Xylit, Sorbit. Die Zucker- oder Zuckeralkoholkomponenten sind bevorzugt feinteilig, mit mittleren Teilchengrößen von 5 bis 100 µm. Gewünschtenfalls können die Teilchengrößen durch Mahlen eingestellt werden. Bevorzugte Teilchengrößen liegen bei 30 bis 50 µm. Es kann sich aber auch empfehlen Teilchengrößen kleiner 30 µm zu verwenden. Ebenso kann es sich empfehlen, Zuckern beziehungsweise Zuckeralkohole einzusetzen, die Mischungen von Fraktionen mit unterschiedlicher Partikelgröße enthalten, bespielsweise Mischungen aus 30 bis 70 Gew.-% einer Korngrößenfraktion mit einer mittleren Teilchengröße von < 30 µm und 30 bis 70 Gew.-% einer Korngrößenfraktion mit einer mittleren Teilchengröße von 30 bis 50 µm. Bevorzugt werden Mannit, Erythrit oder Mischungen davon eingesetzt.

Als Komponentea2) werden Sprengmittel in Mengen von 1 bis 25 Gew.-%, bevorzugt, 2 bis 15 Gew.-%, besonders bevorzugt 3 bis 10 Gew.-%, eingesetzt. Solche Sprengmittel sind wasserunlöslich, aber nicht filmbildend. Als Sprengmittel eignet sich quervernetztes Polyvinylpyrrolidon, Croscarmellose, eine quervernetzte Carboxymethylcellulose, wobei als Croscarmellose erfindungsgemäß auch deren Natrium- und Calciumsalze gemeint sind. Weiterhin eignet sich Natriumcarboxymethylstärke. Ebenso eignet sich L-Hydroxypropylcellulose, bevorzugt mit 5 bis 16 % Hydroxypropoxy-gruppen, wie in USP/NF 2005 beschrieben.

Als Komponentea3) werden wasserunlösliche Polymere eingesetzt in Mengen von 1 bis 15 Gew.-%, bevorzugt 1 bis 10 Gew.-%, eingesetzt. Dabei handelt es sich um Polymere , . Bevorzugt sind Polymere, die im pH-Bereich von 1 bis 14 unlöslich sind, also eine bei jedem pH-Wert pH-unabhängige Wasserunlöslichkeit aufweisen. Weiterhin eignen sich aber auch Polymere, die bei jedem pH-Wert im pH-Bereich von 6 bis 14 wasserunlöslich sind.

Die Polymere sollen filmbildende Polymere sein. Filmbildend bedeutet in diesem Zusammenhang, dass die Polymere in wässriger Dispersion eine Mindestfilmbildetemperatur von -20 bis +150 °C, bevorzugt 0 bis 100 °C aufweisen.

Geeignete Polymere sind Polyvinylacetat, Ethylcellulose, Methylmethacrylat-Ethylacrylat-Copolymere, Ethylacrylat-Methylmethacrylat-Trimethylammoniumethylmethacrylat-Terpolymere. Butylmethacrylat-Methylmethacrylat-Dimethylaminoethylmethacrylat-Terpolymere
Die Acrylat-Methacrylat-Copolymere sind näher beschrieben in der Europäischen Pharmacopoeia als Polyacrylate Dispersion 30%, in der USP als Ammonio Methacrylate Copolymer und in JPE als Aminoalkyl-Methacrylate Copolymer E.
Als bevorzugte Komponente c) kommt Polyvinylacetat zum Einsatz. Dieses kann als wässrige Dispersion mit Feststoffgehalten von 10 bis 45 Gew.-% eingesetzt werden. Bevorzugt ist zudem Polyvinylacetat mit einem Molekulargewicht zwischen 100.000 und 1.000.000 Dalton besonders bevorzugt zwischen 200.000 und 800.000 Dalton. Weiterhin können die Formulierungen als Komponentena4) wasserlösliche Polymere in Mengen von 0 bis 15 Gew.-% enthalten. Geeignete wasserlösliche Polymere sind beispielsweise Polyvinylpyrrolidone, Vinylpyrrolidon-Vinylacetat-Copolymere, Polyvinylalkohole, Polyvinylalkohol-Polyethylenglykol-Pfropfcopolymere, Polyethylenglykole, Ethylenglykol-Propylenglykol-Blockcopolymere, Hydroxypropylmethylcellulose, Hydroxypropylcellulose, Hydroxyethylcellulose, Carragenane, Pektine, Xanthane, Alginate.

Gewünschtenfalls können durch Zusatz von pharmazeutisch üblichen Hilfsstoffen (Komponenten a5)) in Mengen von 0 bis 15 Gew.-%, beispielsweise wie Säuerungsmitteln, Puffersubstanzen, Süßstoffen, Aromen, Geschmacksverstärkern und Farbstoffen Geschmack und Aussehen der aus den Formulierungen erhaltenen Tabletten weiter verbessert werden. Folgende Stoffe sind hierbei besonders geeignet: Citronensäure, Weinsäure, Ascorbinsäure, Natriumdihydrogenphosphat, Cyclamat, Saccharin-Na, Aspartam, Menthol, Pfefferminzaroma, Fruchtaromen, Vanillearoma, Glutamat, Riboflavin, Betacarotin, wasserlösliche Farbstoffe, feinteilige Farblacke.
Durch Zusatz von Verdickungsmitteln wie hochmolekularen Polysacchariden kann das Mundgefühl durch Erhöhung der Weichheit und des Volumengefühls zusätzlich verbessert werden.
Weiterhin können als Komponenten a5) auch Tenside zugegeben werden. Als Tenside eignen sich beispielsweise Natriumlaurylsulfat, Dioctylsulfosuccinat, alkoxilierte Sorbitanester wie Polysorbat 80, polyalkoxilierte Derivate von Rizinusöl oder hydriertem Rizinusöl, beispielsweise Cremophor® RH 40, alkoxilierte Fettsäuren, alkoxilierte Hydroxyfettsäuren, alkoxilierte Fettalkohole, Alkalisalze von Fettsäuren und Lecithine.

Weiterhin können zur weiteren Verbesserung des Zerfalls auch feinteilige Pigmente zugegeben werden, weil sie die inneren Grenzflächen erhöhen und somit Wasser schneller in die Tablette eindringen kann. Diese Pigmente wie Eisenoxide, Titandioxid, kolloidale oder gefällte Kieselsäure, Calciumcarbonate, Calciumphosphate müssen natürlich sehr feinteilig sein, ansonsten entsteht wiederum ein körniger Geschmack.

Die erfindungsgemäß eingesetzten Agglomerate A) eignen sich grundsätzlich zur Verarbeitung mit allen Wirkstoffen B). Insbesondere eignen sich die Granulate für die Herstellung von Arzneiformen mit der im Folgenden genannten Dosierung des jeweiligen Wirkstoffs, wobei der Wirkstoff auch geschmacksmaskiert eingesetzt werden kann: Zolmitriptan 2,5 mg, Rizatriptan 5 mg, Diphenhydramin-HCI (geschmacksmaskiert) 20 mg, Brompheniramin 5 mg, Chlorpheniramin 5 mg, Pseudoephedrin (geschmacksmaskiert) 30mg,
Paracetamol (geschmacksmaskiert) 250 mg, Ibuprofen (geschmacksmaskiert) 200 mg, Acetylsalicylsäure 250 mg (geschmacksmaskiert), Hyoscyaminsulfat 0,125 mg, Mirtazapin 15 mg,
Selegelin-HCI 1,25 mg, Ondansetron 4 mg, Olanzapin 5 mg, Clonazepam 1 mg, Cetirizindihydrochlorid 10 mg, Desloratadin 5 mg, Enalaprilmaleat 5 mg, Domperidonmaleat 10 mg, Scopolamin 0,25 mg,
Oxazepam 15 mg, Lorazepam 2,5 mg, Clozapin 25 mg, Dihydroergotaminmesylat 5 mg,
Nicergolin 5 mg, Phloroglucinol 80 mg, Metopimazin 7,5 mg, Triazolam 0,5 mg, Protizolam 0,5 mg,
Tramadol 50 mg, Zolpidemtartrat 5 mg, Cisapride, Risperidon 2 mg, Azithromycin 100 mg (geschmacksmaskiert), Roxithromycin 50 mg (geschmacksmaskiert), Clarithromycin 125 mg (geschmacksmaskiert), Erythromycinestolat 250 mg (geschmacksmaskiert),
Apomorphin 20 mg, Fentanyl 0,6 mg,

Weiterhin können die Kompaktate als Komponente C) ein Schmiermittel in Mengen von 0 bis 10 Gew.-%, bevorzugt 0,2 bis 5 Gew.-% enthalten. Als Schmiermittel eignen sich Magnesiumstearat, Calciumstearat, Aluminiumstearat, Stearinsäure, Stearylalkohol, Cetylalkohol, Glycerolmonostearat, Glycerinmonobehenat, Glycerolpalmitostearat, Palmitinsäure, Myristinsäure, Beheninsäure, Caprinsäure, Myristylalkohol, Polyethylenglykol, Poloxamere, Talkum oder Natriumbenzoat. Weitere verwendbare Schmiermittel sind in dem Buch "Die Tablette", W. Ritschel und A. Bauer-Brandl, 2. Auflage, 2002, Editio Cantor Verlag Aulendorf angegeben.

Weiterhin können als Komponenten D auch weitere pharmazeutische Hilfsstoffe in Mengen von 0 Gew.-% bis 30.Gew,-%, bevorzugt 0 bis 15 Gew.-%, zu der Kompaktiermischung gegeben werden. Als Komponente D sind die unter a1) bis a5) aufgeführten Stoffe geeignet, ebenso Füllstoffe wie mikrokristalline Cellulose, Stärke oder abgebaute oder modifizierte Stärke. So können auch Farbstoffe, Süßstoffe , Aromen, weitere Sprengmittel, Carbonate, Bicarbonate, Säuerungsmittel oder weitere Hilfsstoffe eingearbeitet werden. Die Verwendung von Farbstoffen, wobei anorganische Pigmente, Organische Farblacke oder wasserlösliche Farbstoffe verwendet werden können, führt beispielsweise zu einheitlich durchgefärbten, schnellzerfallenden Tabletten. Geeignete Farbstoffe sind z. B. Riboflavin, Betacarotin, Anthocyane, Carmin, Indigocarmin, Gelborange S, Chinolingelb, Indigotinlack, Brilliant Blau, Sunset Yellow. Diese weiteren Stoffe können in fester Form zu gegeben werden. Gemäß einer bevorzugten Ausführungsform der Erfindung werden als Komponenten D) weitere Sprengmittel zugegeben.

Die Herstellung der erfindungsgemäß eingesetzten Formulierungen kann durch Aufbau-Agglomeration in Mischern, Wirbelschichtgeräten oder Sprühtürmen erfolgen. Dabei werden feste Ausgangsmaterialien und Granulierflüssigkeit zunächst miteinander vermischt und das feuchte Mischgut anschliessend getrocknet. Gemäß der vorliegenden Erfindung wird als Granulierflüssigkeit eine wässrige Dispersion der Komponente c), des wasserunlöslichen Polymers, eingesetzt.

Bei der Agglomeration in der Wirbelschicht wird eine wässrige Dispersion des wasserunlöslichen Polymers auf eine wirbelnde Mischung aus Zucker oder Zuckeralkohol und quervernetztem PVP gesprüht, wodurch die feinen Teilchen agglomerieren. Die Zulufttemperaturen betragen 30 bis 100°C, die Ablufttemperaturen 20 bis 70°C.

Bei der Herstellung in Sprühtürmen wird vorzugsweise die sogenannte FSD- oder SBD-Technologie (FSD: Fluidized spray drying; SBD: Spray bed drying) eingesetzt. Hierbei wird eine Lösung des Zuckers oder Zuckeralkohols in Wasser zunächst sprühgetrocknet, im unteren Teil des Sprühtrockners oder in einem angeschlossenen Wirbelbett erfolgt die Zugabe von quervernetztem PVP und die Eindüsung einer wässrigen Dispersion des wasserunlöslichen Polymeren, wodurch die Teilchen agglomerieren. Feine Teilchen können ferner nochmals vor die Sprühdüse der Zucker oder Zuckeralkohollösung geblasen werden und zusätzlich agglomeriert werden. Es ist im Sprühturm, FSD oder SBD auch eine Prozeßführung ausgehend von der kristallinen Form des Zuckers oder Zuckeralkohols möglich. Dabei wird der kristalline Zucker oder Zuckeralkohol am Kopf des Sprühturms oder in den Feingut-Recyclingstrom zugegeben. Durch das Versprühen einer wässrigen Dispersion des wasserunlöslichen Polymeren wird dieser kristalline Feststoff im Turm agglomeriert.

Für den Agglomerationsprozess kann es sich als günstig erweisen, einen mehrstufigen Sprühprozess zu fahren. Zu Anfang wird die Sprührate niedrig gehalten, um ein Überfeuchten der Produktvorlage und damit deren Verkleben zu verhindern. Mit zunehmender Prozessdauer kann die Sprührate erhöht und damit die Agglomerationstendenz erhöht werden. Es ist ebenfalls möglich die Zuluftmenge und/oder-temperatur in entsprechender Weise während des Prozesses anzupassen. Besonders während der Trocknungsphase ist es vorteilhaft, die Zuluftmenge zu reduzieren und damit einem Abrieb der Agglomerate durch eine hohe mechanischen Belastung vorzubeugen.

Als weitere Anpassungsparameter für die Agglomeratgröße kann die Feinheit des Sprühtröpfchens der Bindemittellösung bzw. -dispersion (einstellbar über den Zerstäubungsgasdruck), die Düsengeometrie und Abstand der Düse zum Produktbett angesehen werden. Je feiner und einheitlicher gesprüht wird, desto feiner und einheitlicher resultieren die Agglomerate. Je weiter die Düse vom Produktbett entfernt ist, desto schlechter ist das Agglomerationsverhalten.

Weiterhin kann die Agglomeration auch in einem Mischer durch eine kontinuierlich geführte Mischaggregation erfolgen. Eine solche kontinuierlich geführte Form der Mischaggregation ist die sogenannte "Schugi-Granulation". Dabei werden in einem kontinuierlich arbeitenden vertikal angeordneten Hochgeschwindigkeitsmischer feste Ausgangsmaterialien und die das wasserunlösliche Polymer enthaltende Granulierflüssigkeit intensiv miteinander vermischt (siehe auch M. Bohnet, "Mechanische Verfahrenstechnik", Wiley VCH Verlag, Weinheim 2004, S. 198 ff.)

Gemäß einer besonderen Ausführungsform wird das Sprengmittel in der wässrigen Dispersion des wasserunlöslichen Polymers suspendiert.
Die so erhaltenen Agglomerate weisen eine mittlere Teilchengröße von 100 - 600 µm, bevorzugt 120 - 500 µm und besonders bevorzugt von 140 - 400 µm auf.
Das wasserunlösliche, filmbildende Polymer dient dabei als Agglomerationsmittel, um die feinen Zucker oder Zuckeralkoholkristalle und die Teilchen von Sprengmittel zu agglomerieren.

Die Agglomerate werden dann zu Kompaktaten verarbeitet.
Das Prinzip der Kompaktierung ist in dem Buch "Die Tablette", W. Ritschel und A. Bauer-Brandl, 2. Auflage, 2002, Editio Cantor Verlag Aulendorf unter dem Kapitel Trockene Granulation beschrieben. Bevorzugt werden sogenannte Walzenkompaktoren eingesetzt, bei denen das Ausgangsmaterial zwischen 2 rotierenden Walzen verdichtet wird und anschließend wieder zerkleinert wird, so dass gröbere Granulatteilchen entstehen. Die Zerkleinerung soll hierbei möglichst schonend erfolgen, damit wenig Feinanteil entsteht. Die anzuwendenden Preßkräfte liegen zwischen 0,5 und 20 kN/cm, vorzugsweise zwischen 1 und 10 kN/cm. Es hat sich als vorteilhaft erwiesen möglichst niedrige Presskräfte anzuwenden, die gerade zu einem stabilen Kompaktat führen, weil dann die Bruchfestigkeit der daraus hergestellten Tabletten am höchsten ist. Bevorzugt erfolgt die Zerkleinerung durch einen Siebgranulator mit Maschenweiten von 0,5 bis 3 mm.

Die resultierenden Kompaktate weisen in der Regel eine mittlere Teilchengröße zwischen 0,2 und 2 mm auf, vorzugsweise zwischen 0,3 und 1 mm.

Die Kompaktate können auf übliche Art und Weise zu Tabletten verarbeitet werden. Hierzu ist in der Regel das Zumischen von weiterem Schmiermittel sinnvoll. In einer besonderen Ausführung wird auch auf dieser Stufe noch zusätzliches Sprengmittel untergemischt. Die Tablettierung erfolgt auf üblichen Rundläuferpressen, wobei sowohl biplane oder gewölbte Formen als auch Oblong oder Football Shape-Formen hergestellt werden können.

Die erfindungsgemäßen Kompaktate können vorteilhaft auch für die Herstellung von Tabletten verwendet werden, die vor der Anwendung in einem Glas Wasser zerfallen gelassen werden. Auch die Herstellung von Tabletten, die intakt geschluckt werden, ist natürlich möglich.

Überraschenderweise wurde gefunden, dass durch die Kompaktierung sehr geringe Standardabweichungen der Tablettenmasse und des Wirkstoffgehaltes erzielt werden bei gleichzeitig schnellem Zerfall der Tablette. Üblicherweise bewirkt die Kompaktierung eine Verlängerung der Zerfallszeit durch Verringerung der Porosität. Wasser kann dann nicht mehr so leicht in die Hohlräume der Tablette eindringen. Dieser Effekt der Zerfallszeitverlängerung wird bei den erfindungsgemäßen Formulierungen nicht oder nur in geringer Ausprägung gefunden.

Das erfindungsgemäße Verfahren ist besonders für niedrig dosierte Arzneistoffe geeignet, da hierbei häufig Probleme mit der Gehaltseinheitlichkeit zu finden sind. Durch die besondere Plastizität der Komponenten a3) und hierbei insbesondere von Polyvinylacetat werden die Wirkstoffkristalle innig mit den Agglomeraten der Komponente A verklebt, so dass eine Entmischung nicht mehr möglich ist. Die Plastizität sorgt zudem dafür, dass dies schon bei niedriger Kompaktierkraft erfolgt, wodurch die Porosität weitgehend erhalten bleibt.

Daher besitzen die erfindungsgemäßen Kompaktate außergewöhnlich gute Tablettiereigenschaften und Verpressbarkeiten, die zu mechanisch sehr stabilen Tabletten führen. Die Bruchfestigkeit der mit Hilfe der erfindungsgemäßen pharmazeutischen Formulierungen erzeugten Tabletten beträgt > 40 N. Häufig liegen die Bruchfestigkeiten oberhalb von 60 N, selbst unter Verwendung schwer pressbarer Wirkstoffe. Die Friabilitäten betragen < 0,5 %. Beschädigungen beim üblichen Tablettenhandling treten somit nicht auf.

### Beispiele

Zunächst wurden Agglomerate wurden in der Wirbelschicht (GPCG 3.1, Glatt) mittels Top- Spray- Verfahren hergestellt: Zuckeralkohol und Sprengmittel wurden vorgelegt und mit wässriger Bindemitteldispersion agglomeriert. Als wässrige Bindemitteldispersion wurde eine kommerziell erhältliche Polyvinylacetatdispersion (Kollicoat® SR30 D, Fa. BASF AG) verwendet.

| | |
|---|---|
| Ansatzgröße: | 1,8 kg |
| Konzentration der Bindemitteldispersion: | 10 Gew.% Feststoff |

Der durch Wirbelschichtagglomeration hergestellte schnellzerfallende Hilfsstoff wurde mit Wirkstoff und Schmiermittel (Mg-stearat), im Falle der Formulierungen C bis E mit weiteren Hilfsstoffen abgemischt und anschließend auf einem Kompaktor (Gerteis Minipactor) weiterverarbeitet.

**Tabelle 1: Prozessparameter Kompaktierung**

| | A - C | D + E |
|---|---|---|
| Ansatzgröße [g] | 1400 | 1400 |
| Presskraft [kN/cm] | 6,0 | 4,5 |
| Spalt [mm] | 1,0 | 1,5 |
| Siebgröße [mm] | 1,25 | 1,25 |

**Tabelle 2: Zusammensetzung der Kompaktate A bis C in Gew.-%**

| | A | B | C | D | E |
|---|---|---|---|---|---|
| Mannitol (d_{0,5}:36µm) | 89,1 | 81,61 | 86,4 | 75,5 | 70,3 |
| Kollidon CL-SF*) | 4,95 | 4,53 | 4,8 | 5,5 | 5,0 |
| Kollicoat SR 30 D (Feststoff) | 4,95 | 4,53 | 4,8 | 3,0 | 4,2 |
| Kollidon CL-SF*) | - | - | 1,0 | | |
| Croscarmellose-Natrium | | | | | 1,5 |
| L-HPC | | | | 2,0 | |
| Mikrokristalline Cellulose (Avicel PH 105) | | | | | 5,0 |
| Sicovit Rot 30 (Eisenoxid) | | | | 1,5 | |
| Riboflavin | | | | | 2,0 |
| Kollidon VA 64 Fine (Copovidon) | | | | | 1,5 |
| Aspartam | | | | 0,5 | 0,5 |
| Pfefferminz-Aroma | | | | 1,0 | 1,0 |
| Loperamid-HCl | - | - | 2,0 | | |
| Coffein (feines Pulver) | - | 8,33 | - | 10,0 | 8,0 |
| Magnesiumstearat | 1,0 | 1,00 | 1,0 | 1,0 | |

| | | | | | |
|---|---|---|---|---|---|
| 5 *) Crospovidon, mittl. Teilchengröße 17 µm | | | | | |

Die so hergestellten Kompaktate wurden mit 0% bis 2 Gew.-% Schmiermittel (Mg-Stearat) abgemischt und anschließend auf einer Exzentertablettenpresse (Korsch XP1) zu Tabletten mit 40-70N Bruchfestigkeit verpresst.

Die Tabletten wurden hinsichtlich Bruchfestigkeit (Tablettentester HT-TMB-CI-12 F, Fa. Kraemer), Zerfallszeit in Phosphatpuffer pH 7,2 (Zerfallstester ZT 74, Erweka) und Freisetzungsgeschwindigkeit in Magensaft (Freisetzungsgerät, Erweka) untersucht.

**Tabelle 3: Tabletteneigenschaften Formulierungen A bis E**

| | Bruchfestigkeit [N] | Friabilität [%] | Zerfallszeit [s] | Freisetzungsgeschwindigkeit [% nach 10min] |
|---|---|---|---|---|
| A | 57 | <0,2 | 57 | - |
| B | 50 | <0,2 | 35 | 100 |
| C | 70 | <0,2 | 51 | 75 |
| D | 53 | <0,2 | 39 | 98 |
| E | 54 | <0,2 | 33 | 97 |

## Patentansprüche

1. Pharmazeutische Formulierung in Form von Kompaktaten enthaltend
A) einen agglomerierten Hilfsstoffanteil aus
a1) 60 - 97 Gew.-% Trehalose, Mannit, Erythrit, Isomalt, Maltit, Lactit, Xylit, Sorbit oder Mischungen davon
a2) 1 - 25 Gew.-% eines Sprengmittels ausgewählt aus der Gruppe beste hend aus Crospovidon, Croscarmellose, Natriumcarboxymethylstärke und L-Hydroxypropylcellulose,
a3) 1 - 15 Gew.-% wasserunlösliche, filmbildende Polymere mit einer Mindestfilmtemperatur in wässriger Dispersion von -20 bis +150°C
a4) 0 - 15Gew.-%wasserlösliche Polymere, und
a5) 0-15Gew.-%weitere pharmazeutisch übliche Hilfsstoffe, wobei die Summe der Komponenten a1) bis a5) 100 Gew.-% beträgt,
B) mindestens einen Wirkstoff.
C) 0 bis 10 Gew.-%, bezogen auf die Gesamtmenge and A) bis D) eines Schmiermittels oder Formtrennmittels, und
D) gebenenfalls weiteren pharmazeutischen Hilfsstoffen.

2. Formulierung nach Anspruch 1 enthaltend die Komponente C in Mengen von 0,2 bis 5 Gew.-%

3. Formulierung nach Anspruch 1 oder 2, enthaltend als Komponente D ein Sprengmittel.

4. Formulierung nach einem der Ansprüche 1 bis 3 enthaltend als Schmiermittel Magnesiumstearat oder Stearinsäure.

5. Formulierung nach einem der Ansprüche 1 bis 4, enthaltend als Zuckeralkohol Mannit oder, Erythrit oder Mischungen davon.

6. Formulierung nach einem der Ansprüche 1 bis 5, enthaltend ein Croscarmellose als Natrium- oder Calcium-Salz.

7. Formulierung nach einem der Ansprüche 1 bis 6, enthaltend eine L-Hydroxypropylcellulose mit 5 bis 16 % Hydroxypropxygruppen.

8. Formulierung nach einem der Ansprüche 1 bis 7, enthaltend als Sprengmittel Crospovidon

9. Formulierung nach einem der Ansprüche 1 bis 8 , enthaltend als wasserunlösliches filmbildendes Polymer Polyvinylacetat mit einer Mindestfilmtemperatur in wässriger Dispersion von -20 bis +150°C.

10. Formulierung nach einem der Ansprüche 1 bis 9 wobei das wasserunlösliche filmbildende Polymer Polyvinylacetat mit einer Mindestfilmtemperatur in wässriger Dispersion von -20 bis +150°C in Form einer wässrigen Dispersion eingesetzt wird.

11. Formulierung nach einem der Ansprüche 1 bis 10, wobei als wasserlösliches Polymer Polyvinylpyrrolidon verwendet wird.

12. Formulierung nach einem der Ansprüche 1 bis 11, wobei als weitere pharmazeutisch übliche Stoffe Säuerungsmittel, Süßstoffe, Aromen, Geschmacksverstärker, Farbstoffe, Verdickungsmittel, Tenside und feinteilige Pigmente verwendet werden.

13. Formulierung nach einem der Ansprüche 1 bis 12, enthaltend Agglomerate A) aus
a1)70-95 Gew.-% Trehalose, Mannit, Erythrit,
Isomalt, Maltit, Lactit, Xylit, Sorbit oder Mischungen
davon
a2) 1 - 15Gew.-% eines Sprengmittels,
a3)1 - 10 Gew.-% wasserunlösliche, filmbildende Polymere mit einer Mindestfilmtemperatur in wässriger Dispersion von -20 bis +150°C
a4) 0 - 2 Gew.-%wasserlösliches Polyvinylpyrrolidon, und
a5)0-15 Gew.-% weitere pharmazeutisch übliche Hilfsstoffe.

14. Formulierung nach einem der Ansprüche 1 bis 12, enthaltend Agglomerate A) aus
a1) 75 - 95 Gew.-% Mannit oder Erythrit oder einer Mischung davon,
a2) 3 - 10Gew.-% eines Sprengmittels ,
a3) 1 - 10 Gew.-% Polyvinylacetat mit einer Mindestfilmtemperatur in wässriger Dispersion von -20 bis +150°C,
a4) 0-2 Gew.-%wasserlösliches Polyvinylpyrrolidon, und
a5) 0 - 15 Gew.-% weitere pharmazeutisch übliche Hilfsstoffe.

15. Formulierung nach einem der Ansprüche 1 bis 14, erhältlich, indem man in ei- nem ersten Verfahrensschritt die einzelnen Komponenten a1) bis a5) agglomeriert und die so erhaltenen Agglomerate a) mit den Komponenten B), C) und D) einer Kompaktierung unterwirft.

16. Tabletten, enthaltend 20 bis 99 Gew.-%, bezogen auf das gesamte Tablettengewicht, einer pharmazeutischen Formulierung gemäß einem der Ansprüche 1 bis 15.

17. Tabletten nach Anspruch 16, enthaltend weitere Hilfsstoffe.

18. Verfahren zur Herstellung einer pharmazeutischen Formulierung in Form von Kompaktaten gemäß einem der Ansprüche 1 bis 15, **dadurch gekennzeichnet, dass** man zunächst
A) einen Hilfsstoffanteil aus
a1) 60 - 97 Gew.-% Trehalose, Mannit, Erythrit, Isomalt, Maltit, Lactit, Xylit, Sorbit oder
Mischungen davon,
a2) 1 - 25 Gew.-% eines Sprengmittels
a3) 1-15 Gew.-% wasserunlösliche, filmbildende Polymere mit einer Mindestfilmtemperatur in wässriger Dispersion von -20 bis +150°C,
a4) 0 bis 2 Gew.- % wasserlösliches Polyvinylpyrrolidon, und
a5) 0 - 15 Gew.-% weitere pharmazeutisch übliche Hilfsstoffe
wobei die Summe der Komponenten a1) bis a5) 100 Gew.-% beträgt, in Gegenwart von Wasser agglomeriert, und anschliessend die so erhaltenen Agglomerate mit B) mindestens einem Wirkstoff, C) gegebenenfalls einem Sprengmittel, und D) gegebenenfalls weiteren pharmazeutischen Hilffstoffen einer Kompaktierung zuführt.

19. Verfahren nach Anspruch 18, dadurch gekennzeichent, dass die Kompaktierung durch Walzenkompaktierung erfolgt.

## Claims

1. A pharmaceutical formulation in the form of compacts comprising
A) an agglomerated excipient content composed of
a1) 60-97% by weight of trehalose, mannitol, erythritol, isomalt, maltitol, lactitol, xylitol, sorbitol or mixtures thereof
a2) 1-25% by weight of a disintegrant, selected from the group consisting of crospovidone, croscarmellose, sodium carboxymethylstarch and L-hydroxypropylcellulose,
a3) 1-15% by weight of water-insoluble, film-forming polymers having a minimum film forming temperature of from -20 to +150°C in aqueous dispersion
a4) 0-15% by weight of water-soluble polymers and
a5) 0-15% by weight of further pharmaceutically customary excipients
the total of the components a1) to a5) being 100% by weight,
B) at least one active ingredient,
C) 0 to 10% by weight, based on the total amount of A) to D), of a lubricant or mold release agent, and
D) optionally, further pharmaceutical excipients.

2. The formulation according to claim 1 comprising component C in amounts of from 0.2 to 5% by weight.

3. The formulation according to claim 1 or 2, comprising a disintegrant as component D.

4. The formulation according to claim 1 comprising magnesium stearate or stearic acid as lubricant.

5. The formulation according to any of claims 1 to 4, comprising mannitol or erythritol or mixtures thereof as sugar alcohol.

6. The formulation according to any of claims 1 to 5, comprising croscarmellose as sodium or calcium salt.

7. The formulation according to any of claims 1 to 6, comprising an L-hydroxypropylcellulose having 5 to 16% hydroxypropoxy groups.

8. The formulation according to any of claims 1 to 7, comprising crospovidone as disintegrant.

9. The formulation according to any of claims 1 to 8, comprising polyvinyl acetate as water-insoluble film-forming polymer having a minimum film forming temperature of from -20 to +150°C in aqueous dispersion.

10. The formulation according to any of claims 1 to 9, where the water-insoluble film-forming polymer polyvinyl acetate having a minimum film forming temperature of from -20 to +150°C in aqueous dispersion is employed in the form of an aqueous dispersion.

11. The formulation according to any of claims 1 to 10, where polyvinylpyrrolidone is used as water-soluble polymer.

12. The formulation according to any of claims 1 to 11, where acidifiers, sweeteners, flavors, flavor enhancers, colorants, thickeners, surfactants and finely divided pigments are used as further pharmaceutically customary substances.

13. The formulation according to any of claims 1 to 12, comprising agglomerates A) composed of
a1) 70-95% by weight of trehalose, mannitol, erythritol, isomalt, maltitol, lactitol, xylitol, sorbitol or mixtures thereof a2) 1-15% by weight of a disintegrant,
a3) 1-10% by weight of water-insoluble, film-forming polymers having a minimum film forming temperature of from -20 to +150°C in aqueous dispersion
a4) 0-2% by weight of water-soluble polyvinylpyrrolidone, and
a5) 0-15% by weight of further pharmaceutically customary excipients.

14. The formulation according to any of claims 1 to 12, comprising agglomerates A) composed of
a1) 75 - 95% by weight of mannitol or erythritol or a mixture thereof,
a2) 3-10% by weight of a disintegrant,
a3) 1-10% by weight of polyvinyl acetate having a minimum film forming temperature of from -20 to +150°C in aqueous dispersion,
a4) 0-2% by weight of water-soluble polyvinylpyrrolidone, and
a5) 0-15% by weight of further pharmaceutically customary excipients.

15. The formulation according to any of claims 1 to 14, obtainable by agglomerating the individual components a1) to a5) in a first process step and subjecting the agglomerates a) thus obtained to a compaction together with components B), C) and D).

16. A tablet comprising from 20 to 99% by weight, based on the total tablet weight, of a pharmaceutical formulation according to any of claims 1 to 15.

17. The tablet according to claim 16, comprising further excipients.

18. A process for producing a pharmaceutical formulation in the form of compacts according to any of claims 1 to 15, which comprises initially agglomerating
A) an excipient content composed of
a1) 60-97% by weight of trehalose, mannitol, erythritol, isomalt, maltitol, lactitol, xylitol, sorbitol or mixtures thereof,
a2) 1-25% by weight of a disintegrant
a3) 1-15% by weight of water-insoluble film-forming polymers having a minimum film forming temperature of from -20 to +150°C in aqueous dispersion,
a4) 0 to 2% by weight of water-soluble polyvinylpyrrolidone, and
a5) 0-15% by weight of further pharmaceutically customary excipients
the total of the components a1) to a5) being 100% by weight,
in the presence of water, and then feeding the agglomerates thus obtained to a compaction together with B) at least one active ingredient, C), optionally, a disintegrant, and D), optionally, further pharmaceutical excipients.

19. The process according to claim 18, wherein the compacting takes place by roll compaction.

## Revendications

1. Formulation pharmaceutique sous la forme de produits compactés, contenant :
A) une fraction d'adjuvant agglomérée, constituée par
a1) 60 à 97 % en poids de tréhalose, de mannitol, d'érythritol, d'isomaltol, de maltitol, de lactitol, de xylitol, de sorbitol ou de mélanges de ceux-ci,
a2) 1 à 25 % en poids d'un désintégrant choisi dans le groupe constitué par la crospovidone, la croscarmellose, le carboxyméthyl-amidon de sodium et la L-hydroxypropylcellulose,
a3) 1 à 15 % en poids de polymères filmogènes insolubles dans l'eau ayant une température filmogène minimale en dispersion aqueuse de -20 à +150 °C,
a4) 0 à 15 % en poids de polymères solubles dans l'eau et
a5) 0 à 15 % en poids d'autres adjuvants pharmaceutiquement usuels,
la somme des composants a1) à a5) étant de 100 % en poids,
B) au moins un agent actif,
C) 0 à 10 % en poids, par rapport à la quantité totale de A) à D), d'un lubrifiant ou d'un agent de démoulage, et
D) éventuellement d'autres adjuvants pharmaceutiques.

2. Formulation selon la revendication 1, contenant le composant C en quantités de 0,2 à 5 % en poids.

3. Formulation selon la revendication 1 ou 2, contenant un désintégrant en tant que composant D.

4. Formulation selon l'une quelconque des revendications 1 à 3, contenant du stéarate de magnésium ou de l'acide stéarique en tant que lubrifiant.

5. Formulation selon l'une quelconque des revendications 1 à 4, contenant du mannitol ou de l'érythritol ou des mélanges de ceux-ci en tant que sucre-alcool.

6. Formulation selon l'une quelconque des revendications 1 à 5, contenant une croscarmellose en tant que sel de sodium ou de calcium.

7. Formulation selon l'une quelconque des revendications 1 à 6, contenant une L-hydroxypropylcellulose qui contient 5 à 16 % de groupes hydroxypropyle.

8. Formulation selon l'une quelconque des revendications 1 à 7, contenant de la crospovidone en tant que désintégrant.

9. Formulation selon l'une quelconque des revendications 1 à 8, contenant du polyacétate de vinyle ayant une température filmogène minimale en dispersion aqueuse de -20 à +150 °C en tant que polymère filmogène insoluble dans l'eau.

10. Formulation selon l'une quelconque des revendications 1 à 9, dans laquelle le polymère filmogène insoluble dans l'eau polyacétate de vinyle ayant une température filmogène minimale en dispersion aqueuse de -20 à +150 °C est utilisé sous la forme d'une dispersion aqueuse.

11. Formulation selon l'une quelconque des revendications 1 à 10, dans laquelle de la polyvinylpyrrolidone est utilisée en tant que polymère soluble dans l'eau.

12. Formulation selon l'une quelconque des revendications 1 à 11, dans laquelle des acidifiants, des édulcorants, des arômes, des exhausteurs de goût, des colorants, des épaississants, des tensioactifs et des pigments finement divisés sont utilisés en tant qu'autres substances pharmaceutiquement usuelles.

13. Formulation selon l'une quelconque des revendications 1 à 12, contenant des agglomérats A) constitués par
a1) 70 à 95 % en poids de tréhalose, de mannitol, d'érythritol, d'isomaltol, de maltitol, de lactitol, de xylitol, de sorbitol ou de mélanges de ceux-ci,
a2) 1 à 15 % en poids d'un désintégrant,
a3) 1 à 10 % en poids de polymères filmogènes insolubles dans l'eau ayant une température filmogène minimale en dispersion aqueuse de -20 à +150 °C,
a4) 0 à 2 % en poids de polyvinylpyrrolidone soluble dans l'eau et
a5) 0 à 15 % en poids d'autres adjuvants pharmaceutiquement usuels.

14. Formulation selon l'une quelconque des revendications 1 à 12, contenant des agglomérats A) constitués par
a1) 75 à 95 % en poids de mannitol ou d'érythritol ou de mélanges de ceux-ci,
a2) 3 à 10 % en poids d'un désintégrant,
a3) 1 à 10 % en poids de polyacétate de vinyle ayant une température filmogène minimale en dispersion aqueuse de -20 à +150 °C,
a4) 0 à 2 % en poids de polyvinylpyrrolidone soluble dans l'eau et
a5) 0 à 15 % en poids d'autres adjuvants pharmaceutiquement usuels.

15. Formulation selon l'une quelconque des revendications 1 à 14, pouvant être obtenue par agglomération, lors d'une première étape de procédé, des composants a1) à a5) individuels et soumission des agglomérats a) ainsi obtenus à un compactage avec les composants B), C) et D).

16. Comprimés, contenant 20 à 99 % en poids, par rapport au poids total des comprimés, d'une formulation pharmaceutique selon l'une quelconque des revendications 1 à 15.

17. Comprimés selon la revendication 16, contenant d'autres adjuvants.

18. Procédé de fabrication d'une formulation pharmaceutique sous la forme de produits compactés selon l'une quelconque des revendications 1 à 15, **caractérisé en ce que**
A) une fraction d'adjuvant, constituée par
a1) 60 à 97 % en poids de tréhalose, de mannitol, d'érythritol, d'isomaltol, de maltitol, de lactitol, de xylitol, de sorbitol ou de mélanges de ceux-ci,
a2) 1 à 25 % en poids d'un désintégrant,
a3) 1 à 15 % en poids de polymères filmogènes insolubles dans l'eau ayant une température filmogène minimale en dispersion aqueuse de -20 à +150 °C,
a4) 0 à 2 % en poids de polyvinylpyrrolidone soluble dans l'eau,
a5) 0 à 15 % en poids d'autres adjuvants pharmaceutiquement usuels,
la somme des composants a1) à a5) étant de 100 % en poids, est tout d'abord agglomérée en présence d'eau, puis les agglomérats ainsi obtenus sont introduits dans un compactage avec B) au moins un agent actif, C) éventuellement un désintégrant et D) éventuellement d'autres adjuvants pharmaceutiques.

19. Procédé selon la revendication 18, **caractérisé en ce que** le compactage a lieu par compactage par cylindres.
